# EUROPEAN PATENT SPECIFICATION

(11) **EP 1 578 763 B1**
(45) Date of publication and mention of the grant of the patent: **07.01.2009**
(21) Application number: 03790033.9
(22) Date of filing: 24.11.2003
(51) Int. Cl.: C12N 15/10, C12N 1/08, C12Q 1/68

(54) **PROCESS FOR PURIFICATION OF PLASMID DNA**
REINIGUNGSVERFAHREN FÜR PLASMID-DNA
PROCEDE DE PURIFICATION D'ADN PLASMIDIQUE

(30) Priority: 23.12.2002 US 435270 P
(43) Date of publication of application: 28.09.2005
(73) Proprietor: VICAL INCORPORATED, San Diego, California 92121-4340 (US)
(72) Inventor: BUDAHAZI, Gregg, San Diego, CA 92109 (US); GOFF, Blake, San Diego, CA 92131 (US)
(74) Representative: Walton, Seán Malcolm
(86) International application number: PCT/US2003/037665
(87) International publication number: WO 2004/060277

(56) References cited:
- EP-A1- 0 964 057
- WO-A-01/46215
- WO-A-96/02658
- WO-A-99/29832
- WO-A2-95/21250
- US-A1- 2001 034 435
- FERREIRA G.N.M. ET AL.: 'Downstream processing of plasmid DNA for gene therapy and DNA vaccine applications' vol. 18, September 2000, pages 380 - 388, XP000972180
- DIOGO M.M. ET AL.: 'Separation and analysis of plasmid denatured forms using hydrophobic interaction chromatography' ANALYTICAL BIOCHEMISTRY vol. 275, November 1999, pages 122 - 124, XP002178574

## Description

### Field of the Invention

The invention relates to a process for purifying plasmid DNA. The invention also relates to a DNA product made by the process of the invention. The DNA product is suitable for pharmaceutical use.

### Related Art

Plasmids are used, e.g., for preparation of antibodies, genetic analyses, screening, and laboratory analyses. Recent advances in biology have provided the possibility of using polynucleotides encoding polyamino acids for treatment of diseases. One method of gene delivery to humans is to use plasmid DNA, a closed circular form of DNA.

In some cases, large quantities of plasmid DNA are required to provide effective treatment to a population of patients. Thus, a process that is scaleable to allow for efficient, cost effective production and shortened process time, and provides high recovery of plasmid DNA is desired.

Further, the plasmid DNA will have to be very highly purified to allow administration of the plasmid DNA to patients. Because the plasmids used in clinical applications are produced typically by bacteria, e.g., *E. coli*, the purification process must efficiently separate bacterial endotoxin, protein, carbohydrate, and genomic (chromosomal) DNA from plasmid DNA, at very large scale. Because chromosomal DNA and plasmid DNA are so similar the process needs to be particularly effective at removing the chromosomal DNA, especially for use of the purified plasmid in pharmaceutical compositions.

Methods for purifying plasmid DNA are provided in, e.g., U.S. Patent Nos. 5,561,064, 6,214,586, and 6,441,160, and U.S. Appl. Pub. No.2001/0034435. However, new and improved methods for purifying plasmid DNA are sought, and which are scaleable to produce large quantities of plasmid DNA of high purity to be used as a therapeutic in mammals such as humans.
WO 01/46215 describes a process directed to purification of DNA from E. *coli* cells. The lysis step in the process disclosed in WO 01/46215 is accomplished by a lysozyme mix and heat exchanger. The process uses cetyltrimethylammonium bromide as a precipitation reagent after the first filtration step.
WO 96/02658 discloses a DNA purification method which uses continuous flow centrifugation and requires RNase treatment.
WO 99/29832 discloses a method for purifying plasmid DNA from genomic DNA for eventual use in humans and requires an overnight mixing step.
US2001/0034435 A1 is drawn to purification of DNA. The method achieves cell lysis by combining resuspended cells (in solution I) with lysis solution II using a single pass through a static mixer, followed by a pass through "lysis coils." The lysate is then dumped into a jacketed chilled neutralization vessel containing a high salt precipitant at neutral to slightly basic pH (1M potassium acetate/7M ammonium acetate pH 7-9) to remove cell debris and other cellular contaminants. This neutralization process step requires at least six hours at 4°C. US2001/0034435 removes cell debris after lysis by centrifugation, followed by an unspecified type of filtration (removal of residual debris or fine particulate matter). After clarification, US2001/0034435 dilutes their sample with WFI (conductivity reduction) and then proceeds directly to anion exchange chromatography. US2001/0034435 uses Fractogel EMD TMAE resin. This is a "tentacle" ion exchanger having a particle size of 20-40 microns. US2001/0034435 states a binding capacity for plasmid DNA of about 3 mg/mL, preferably 1.5 mg/mL of resin and supercoiled DNA enrichment of at least 80%.

### SUMMARY OF THE INVENTION

The invention is directed to a process for purifying plasmid DNA from host cell impurities to obtain a DNA product, the process comprising: (a) lysing host cells containing the plasmid DNA to obtain a lysate; (b) clarifying the lysate by depth filtration to obtain a clarified lysate; (c) ultrafiltering the clarified lysate to obtain an ultrafiltered clarified lysate; (d) adding a first precipitating agent in sufficient quantity to the ultrafiltered clarified lysate to obtain a precipitate of the plasmid DNA; (e) dissolving the precipitate to obtain a first solution; (f) adding a second precipitation agent in sufficient quantity to the solution to precipitate the host cell impurities and to obtain a solute containing the plasmid DNA; (g) transferring the solute into another buffer to obtain a second solution; (h) applying the second solution to an anion exchange chromatography (AEX) material to obtain an eluate containing the plasmid DNA; and (i) applying the eluate to a hydrophobic interaction chromatography (HIC) material to obtain the DNA product.

In one embodiment of the invention, the AEX chromatography material comprises a ceramic matrix. The average particle diameter of the ceramic matrix for the AEX support can be about 10 µm to about 200 µm. The average pore size of the ceramic matrix for the AEX support can be about 750 Å to about 2000 Å. In the AEX chromatography material comprising a ceramic matrix, the average pore size of the AEX chromatography material can be about 10 Å to about 100 Å.

In some embodiments of the invention, the average particle diameter of resin for the HIC chromatography material can be about 50 µm to about 150 µm. The average pore size of resin for the HIC chromatography material can be about 25 nm to about 100 nm.

In the invention, the lysing in (a) can be by alkaline lysis. The clarifying in (b) can be by diatomite aided depth filtration. The ultrafiltering in (c) can be by hollow fiber ultrafiltration. The first precipitating agent in (d) can be polyethylene glycol (PEG). The second precipitating agent in (f) can be ammonium acetate. The eluate in (h) can be adjusted to a concentration of about 1 M to about 2 M ammonium sulfate. The HIC chromatography material in (i) can contain cross-linked agarose resin. The DNA product in (i) can be further concentrated by ultrafiltration. The DNA product in (i) can be further diafiltered to remove ammonium sulfate. The DNA product can be further precipitated with ethanol.

In certain embodiments, the process can be conducted in the absence of any added enzymes, organic extractants, or mutagenic reagents.

The DNA product of the invention can be further sterilized, formulated, and filled in a sterile container.

In the invention, the host cells can be bacteria.

The invention is further directed to a DNA product obtained by the process of the invention. The DNA product can comprise about 95% or greater by weight of circular plasmid DNA. The DNA product can comprise less than about 5% by weight of RNA. The DNA product can comprise less than about 0.002 µg of host DNA/µg of DNA product. The DNA product can comprise less than about 0.001 µg of protein/µg of DNA product. The DNA product can comprise less than about 0.01 EU/µg of DNA product.

The invention is also directed to a DNA product comprising about 95% or greater by weight of circular plasmid DNA, wherein the DNA product contains less than about 5% by weight of RNA, less than about 0.002 µg of host DNA/µg of DNA product, less than about 0.001 µg of protein/µg of DNA product, and less than about 0.01 EU/µg of DNA product.

The invention is also directed to a medicament comprising the DNA product of the invention. In one embodiment, the invention is directed to a sterile container comprising the DNA product. In another embodiment, the invention is directed to a kit comprising the DNA product. The DNA product can be formulated with pharmaceutically inactive agents or pharmaceutically active agents. Such formulations are well within the skill of the art.

### BRIEF DESCRIPTION OF THE DRAWING

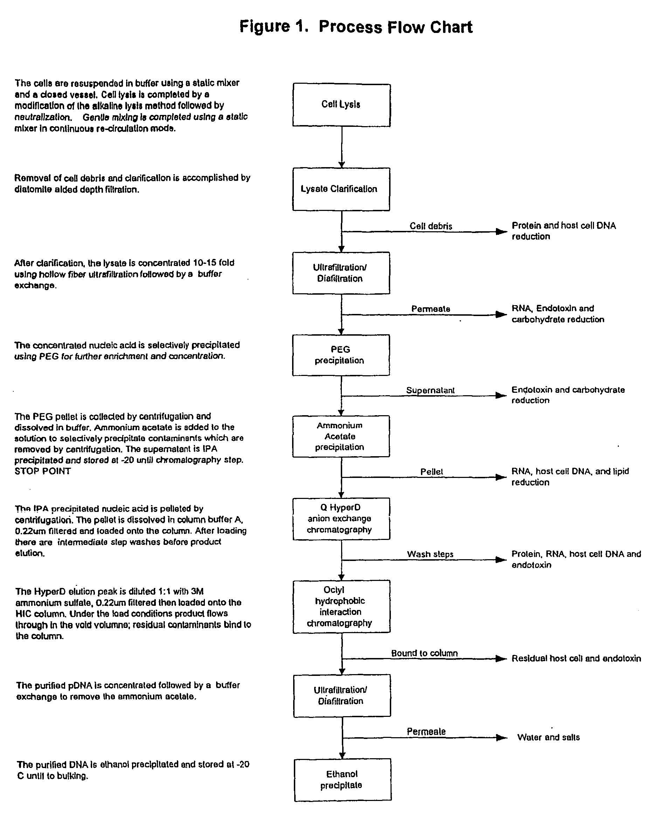

FIG. 1. A process flow chart of one embodiment of the invention.

### DETAILED DESCRIPTION OF THE INVENTION

The invention is directed to a process for purifying plasmid DNA from host cell impurities to obtain a DNA product, the process comprising: (a) lysing host cells containing the plasmid DNA to obtain a lysate; (b) clarifying the lysate by depth filtration to obtain a clarified lysate; (c) ultrafiltering the clarified lysate to obtain an ultrafiltered clarified lysate; (d) adding a first precipitating agent in sufficient quantity to the ultrafiltered clarified lysate to obtain a precipitate of the plasmid DNA; (e) dissolving the precipitate to obtain a first solution; (f) adding a second precipitation agent in sufficient quantity to the solution to precipitate the host cell impurities and to obtain a solute containing the plasmid DNA; (g) transferring the solute into another buffer to obtain a second solution; (h) applying the second solution to an anion exchange chromatography (AEX) material to obtain an eluate containing the plasmid DNA; and (i) applying the eluate to a hydrophobic interaction chromatography (HIC) material to obtain the DNA product.

The DNA product of the process of the invention is suitable for, but not limited to, preparation of antibodies, genetic analyses, screening, laboratory analyses, and for manufacture of pharmaceutical grade DNA formulation for uses such as medicaments. The process yields a high quality DNA product. The DNA product produced by this manufacturing process is essentially free of, contains only trace levels of, or contains only undetectable levels of host cell derived impurities. Such host cell impurities include, but are not limited to, RNA, protein, endotoxins, pyrogens and host chromosomal DNA. In the process of the invention, the steps can implement salts, buffers, solvents, extractants and precipitating agents that are recognized as safe by the Food and Drug Administration (FDA) and other like international regulatory agencies. The FDA regulates food additives through the GRAS list. Food additives are substances that are added to foods but are not normally consumed by themselves as foods. GRAS is an acronym for "generally recognized as safe." The GRAS list is established by the FDA and consists of substances that have been determined to be safe through either scientific testing, or extensive usage dating before 1958.

The process is composed of scalable unit operations. The process allows production of milligram, gram and kilogram quantities of plasmid DNA.

Following purification, the DNA product can be analyzed to ensure that it meets specifications for pharmaceutical grade DNA product, which can be used as a medicament. The DNA product can be formulated for sterile container fill. The sterile container containing the DNA product of the invention can meet the quality criteria (identity, purity, and potency) required for a pharmaceutical product.

Although the DNA product of the invention is not limited to pharmaceutical use, the product is suitable for pharmaceutical use as the process of the invention can provide pharmaceutical grade DNA product. By pharmaceutical grade is intended the standards set by FDA and other like international regulatory agencies. Federal law requires that the use of pharmaceutical agents in the therapy of humans be approved by an agency of the Federal government. Responsibility for enforcement is the responsibility of the FDA, which issues appropriate regulations for securing such approval, detailed in 21 U.S.C. 301-392. Regulation for biologic material, comprising products made from the tissues of animals is provided under 42 U.S.C. 262. Most foreign countries require similar approval. Regulations vary from country to country, but the individual procedures are well known to those in the art.

### Plasmids and Host Cells

Plasmid DNAs that can be purified according to the process of the present invention are not limited. Plasmids include, e.g., prokaryotic and eukaryotic vectors, cloning and expression vectors, pBR322 and pUC vectors and their derivatives. To incorporate various origins of replication, plasmids can contain, e.g., prokaryotic origins of replication, such as pMB1 and ColE1, or eukaryotic origins of replication, such as those facilitating replication in yeast, fungi, insect, and mammalian cells (e.g., SV40 ori). To encompass numerous genetic elements to facilitate cloning and expression, plasmids can contain, e.g., selectable genes, polylinkers, promoters, enhancers, leader peptide sequences, introns, polyadenylation signals, or combinations thereof. The selection of vectors, origins, and genetic elements will vary based on requirements and is well within the skill of workers in this art.

The plasmid can include additional genetic elements as desired. For example, the plasmid can include a polynucleotide encoding a selectable marker, such as an antibiotic resistance gene, e.g., puromycin resistance, neomycin resistance, hygromycin resistance, and thymidine kinase resistance.

The plasmid can include a polylinker that includes any of a variety of restriction endonuclease recognition sites to facilitate cloning. Such a polylinker can be positioned, for example, downstream of the second promoter to permit the second promoter to be operably linked to a polynucleotide that is cloned into the polylinker. Accordingly, the plasmid can also include a polynucleotide encoding a polypeptide, wherein the polynucleotide (i.e., a transgene) is operably linked to a promoter.

The polynucleotide can encode any of a variety of polypeptides, such as, without limitation, erythropoietin, adenosine deaminase, Factor VIII, Factor IX, dystrophin, β-globin, LDL receptor, CFTR, insulin, an anti-angiogenesis factor, a growth hormone, α1-antitrypsin, phenylalanine hydroxylase, tyrosine hydroxylase, ornithine transcarbamylase, arginosuccinate synthetase, UDP-glucuronysyl transferase, an interleukin, and an interferon.

As an alternative to using a polynucleotide that encodes a polypeptide, the polynucleotide can encode an antisense RNA. Such an antisense RNA which can be used to inhibit expression of a target gene, *e.g*., where the gene expression is undesirable (*e.g*., the gene of a pathogen) or where the level of gene expression is undesirably high in a cell. The encoded antisense RNA can function to inhibit gene expression in any of a variety of ways, *e.g*., by interfering with replication and/or transcription. The functions of RNA to be interfered with include any vital function such as, for example, translocation of the RNA to the site of protein translation, translation of protein from the RNA, splicing of the RNA to yield one or more mRNA species, and catalytic activity which may be engaged in or facilitated by the RNA. For example, the antisense RNA can hybridize to a regulatory element (*e.g*., transcription start site) and/or all or a portion of a coding sequence of a gene to inhibit expression of that gene. Examples of intragenic regions that can be targeted by antisense RNAs include the region of the translational start site and the termination codon of the open reading frame of the gene, both of which can readily be identified using conventional principles of molecular biology. Other targets include the junctions between introns and exons. The overall effect of such interference with target nucleic acid function is modulation of the expression of the target gene. The antisense RNAs typically are at least about 8 nucleotides in length, e.g., at least about 10, 12, 15, 18, 20, 25 nucleotides, and generally, antisense RNAs that are up to about 30, 50, or 100 nucleotides in length are of sufficient length (*e.g*., about 8-100 nt, about 10-15 nt, about or 18-30 nt).

The host or host cell containing the plasmid includes, but is not limited to, prokaryotes and eukaryotes, including bacterial, yeast, fungi, insect and mammalian cells. Hosts include microbial cells, especially microorganisms like *E. coli*. Any suitable strain of *E. coli* is contemplated. Likewise, genes encoding diverse structural proteins (or peptides, polypeptides, glycoproteins, phosphoproteins, amidated proteins, etc.) can be inserted into the plasmid, which genes can constitute genomic DNA, cDNA, synthetic DNA, polynucleotide and oligonucleotide, etc. These nucleic acids can be obtained using chemical synthesis or gene manipulation techniques (see Sambrook, Fritsch, Maniatis, Molecular Cloning, A Laboratory Manual, Second Edition, Cold Spring Harbor Laboratory Press, 1989; and Current Protocols in Molecular Biology, Greene Publishing Assoc. & Wiley, 1987) and, further, can be inserted into plasmids, and the plasmids subsequently introduced into host cells using additional gene manipulation techniques (*id*.).

Introduction of the plasmid into the host cell can be effected by heat shock, calcium phosphate transfection, DEAE-Dextran mediated transfection, or electroporation (Hanahan, D., J. Mol. Biol. 166:557-580 (1983); Davis, L. et al., Basic Methods in Molecular Biology (1986)).

The plasmid can encode any of a variety of polypeptides such as, without limitation, erythropoietin (*e.g*., for treating anemia), adenosine deaminase, Factor VIII, Factor IX, dystrophin, β-globin, LDL receptor, CFTR, insulin, anti-angiogenesis factors, growth hormones, α1-antitrypsin, phenylalanine hydroxylase, tyrosine hydroxylase, ornithine transcarbamylase, arginosuccinate synthetase, UDP-glucuronysyl transferase, interleukins, and interferons.

Culturing of a plasmid DNA-containing host can be carried out using known processes such as those disclosed in the aforementioned documents, and include, but is not limited to, the use of incubator, bioreactor, fermentor etc., according to batch fermentation, fed batch fermentation, continuous culture, Type I, II and III fermentation, aseptic fermentation, consortium fermentation, protected fermentation, etc. Fitting the conditions (e.g., medium, temperature, pH, hours, agitation, aeration, etc.) for culture to the circumstances is empirical and well within the skill of those in the art.

### Cell Paste

After culturing of the plasmid DNA-containing host cells, the host cells are collected, e.g., by laboratory, pilot plant, or industrial scale centrifugation and/or filtration, including micro- and/or ultra-filtration, depending on the specific application. The collected cells can be stored frozen, using standard methodology, or can be processed immediately for purification of the plasmid DNA. An embodiment of the process of the invention is provided as a process flow chart in Figure 1.

### Resuspension of Cells in Buffer

The cells are resuspended in buffer using resuspension techniques. The buffer is not limited and can include a reagent that is "generally recognized as safe" (GRAS) by the FDA. Such buffers include Tris-HCl, sodium acetate, potassium citrate, sodium phosphate monobasic or dibasic or a mixture, and the like. The selection of pH and ionic strength is well within the level of skill in the art.

In some embodiments, resuspension can be achieved using a static mixer and a closed vessel, which can result in: (1) better cell resuspension and mixing of solutions; (2) better control of lysis times; and (3) lower shear, proving less chromosomal DNA contamination.

### Cell Lysis

The cells are lysed using lysis techniques, to liberate DNA from the cells. In some embodiments, the cells are lysed without resort to animal- or otherwise-derived enzymes, such as lysozyme. Lysis can be carried out in a dilute base or a dilute base and a detergent, which can be GRAS reagents. Such bases include, but are not limited to, sodium hydroxide, potassium hydroxide, sodium bicarbonate. Such detergents include, but are not limited to, pharmaceutically acceptable nonionic surfactants, e.g., sodium dodecyl sulfate (SDS), polysorbates (sold under the trademark Tween®), polyoxyethylene ethers (sold by Union Carbide as Triton®), and the like. A NaOH and SDS combination is advantageous. Optimization of pH and ionic strength is well within the skill of the art.

In some embodiments, gentle mixing is provided during lysis using a static mixer in continuous re-circulation mode.

An alternative to alkaline lysis can be mechanical breakage using, e.g., a French Press, a microfluidizer, sonication, and the like.

Precipitation techniques can be used to precipitate non-plasmid or host cell impurities such as chromosomal DNA, high molecular weight RNA, protein and membrane complexes. Also, the lysate can be acidified at this stage, using acidification techniques, to facilitate subsequent removal of insoluble material resulting from cell lysis by reducing the viscosity created during alkaline treatment. Any acid can be employed. For pharmaceutical use, it can be a GRAS ingredient, such as glacial acetic acid, phosphoric acid, citric acid, and the like. The matching of pH and ionic strength is empirical and well within the skill of art.

Some embodiments of the invention involve the use of a modified, non-acidic neutralization solution that can be added to an alkaline/detergent lysate to precipitate RNA as well as chromosomal DNA, protein, lipids, etc. from the lysate. This can result in: (1) removal of majority of RNA (especially high molecular weight) prior to column chromatography; (2) elimination of exposure of plasmid DNA to damaging acid; and (3) elimination of need to partially purify plasmid prior to RNA precipitation. Neutralization can be achieved with sodium acetate, sodium hydrogen sulfate, sodium citrate, or the base of any weak acid. In some embodiments, neutralization is achieved by adding 3M potassium acetate, pH 5.5, while recirculating through the static mixer.

### Removal of Cell Debris and Other Impurities

The cell debris and high molecular weight impurities released during cell lysis are removed by laboratory, pilot plant or industrial scale centrifugation and/or filtration. Filtration ensures a clarified lysate prior to the next positive precipitation. As is well known to practitioners, filtration can be facilitated to enhance the flow properties of heterogeneous or viscous solutions by the employment of aids such as cellulose, siliceous earth, Celite® filtering aid, etc.

The material through which the lysate is filtered according to the process of the invention comprises apertures or pores having a diameter large enough to allow plasmid DNA to pass through with the filtrate but small enough so that the assembled filter retains a large proportion of the insoluble material (depth filtration). Pore sizes can be irregular and variable as long as the total effect is to retain the precipitate. Accordingly pore sizes can be from about 0.1 µm to about 100 µm, or from about 0.5 µm to about 50 µm. The material from which the filter is manufactured can be a synthetic material or an organic or inorganic natural material. The filter material is also advantageously autoclavable, malleable, and strong, and is capable of being assembled in layers to achieve various filtering efficiencies adaptable to unique experimental situations. It can be non-absorbent, and can be hydrophilic or hydrophobic; however, the hydrophobicity should not retard the flow of filtrate. Examples of suitable filter materials are diatomite (skeletal remains of minute algae (diatoms) found in marine deposits that have lifted above sea level), glass, plastic, or metal screens, porous films, cellulose or fiber mats, woven or nonwoven fabrics, or a synthetic fabric, such as nylon or rayon, e.g., Miracloth® (Calbiochem, Cat. #475855, La Jolla, CA), a synthetic nonwoven rayon fabric having an average pore size of about 22 to about 25 µm. The filtration can be performed under conditions of centrifugal force, pressure or vacuum. In some embodiments, diatomite aided depth filtration is used for clarification of the lysate and removal of cell debris, e.g., removal or reduction of host cell DNA and protein. In some embodiments, pressure fed diatomite aided depth filtration with a depth filter of about 0.45 µm (nominal) pore size is used.

Centrifugation can be used as another means of removing cell debris and other impurities. After cell lysis, the material can be spun at a sufficient g-force and time period to permit complete recovery of material. G-forces of about 2000 x g to about 6500 x g can be used and time periods from about 30 to about 60 minutes can be used. When the centrifugation is completed, the supernatant is decanted and saved for further processing steps.

The clarified filtrate (or supernatant) undergoes a volume reduction of about 10 to about 15 volumes followed by a buffer exchange of about 4 to about 10 volumes using ultrafiltration and diafiltration (UF/DF). The ultrafiltration membrane can be cleanable and chemically compatible with the solutions/buffers used. A number of suitable membrane materials are available including cellulose acetate, polysulfone, polyethersulfone or polyvinylidene difluoride; Polysulfone is one membrane material that can be used in this process. Many types of ultrafiltration devices are available including e.g., flat sheet, spiral wound, hollow-fiber or stir cell. Hollow fiber tangential flow filtration (TFF) can be used in this process. Membrane pore selection will depend on the membrane manufacturer, DNA size and conformation. Molecular weight cut-off (MWCO) from about 50 KD to about 500 KD can be used; a 300 KD membrane can be used in this process. The hollow fibers retain the plasmid DNA (retentate) while impurities, water and salts flow through the membrane (permeate). The UF/DF process step results in the removal or reduction of RNA, endotoxin and carbohydrate.

### Plasmid DNA from Clarified Filtrate

Polyethylene glycols (PEGs) or other similar high molecular weight polyols can be used as precipitants and extractants in both protein and nucleic acid recovery. PEG precipitation is advantageous for the manufacture of DNA. It is an acceptable ingredient in pharmaceuticals. In this step, a PEG is added to the clarified filtrate to result in a high concentration of PEG. Bringing the final concentration to about 8% PEG to about 12% PEG, e.g., about 10% PEG (weight/volume) can be advantageous. Typically a PEG-8000 can be used. However, other PEGs of variety of molecular weights can be use, such as, but not limited to, PEG-3000 to PEG-20000. Application of standard precipitation techniques and selection of pH and ionic strength is well within the skill of those in the art.

### Collecting Partially Purified DNA

The precipitate of plasmid DNA that is formed as a consequence of alcohol or PEG precipitation is separated in the usual way by laboratory, pilot plant, or industrial scale centrifugation and/or filtration. The precipitation permits further removal or reduction of endotoxin and carbohydrate which are retained in the supernatant.

Centrifugation is used as one means of collecting the PEG precipitant. The material can be spun at a sufficient g-force and time period to permit complete recovery of material. For batch centrifugation the PEG precipitated DNA can be transferred to appropriate size centrifuge bottles. G-forces of about 2000 x g to about 6500 x g can be used and time periods from about 30 to about 60 minutes can be used. When the centrifugation is completed, the supernatant is decanted and discarded.

### Dissolving Partially Purified DNA in Buffer and Precipitation of Impurities

When the plasmid precipitate is resuspended in a buffer, resuspension techniques known in the art can be used, and buffers that are, but not limited to, GRAS buffering agents can be employed.

In this step, high salt precipitation can be performed using precipitation techniques and selecting pH and ionic concentrations as will be known to those with skill in the art. A high salt precipitation is effective to remove some of the contaminating RNA and much of the lipopolysaccharides, and further reduce host cell DNA. The type of salt is not limited, but can be a GRAS ingredient. Ammonium acetate, lithium chloride, sodium chloride, sodium acetate, etc., are useful.

### Removal of Precipitated Impurities

The precipitate of impurities that is formed as a consequence of high salt precipitation is separated using standard techniques by laboratory, pilot plant or industrial scale centrifugation and/or filtration. Filtration can be carried out on any filter material that meets the requirements of a pore size that is effective to retain the precipitate of impurities, while allowing a plasmid-DNA filtrate to be recovered. Effective filtration is characterized by maximum recovery of contaminants and minimum retention of plasmid on the filter, that is, optimal flow-through of product. Suitable filters can have pore diameters or ranges of pore diameters from about 0.1 µm to about 100 µm, or from about 0.1 µm to about 50 µm. The filter material for this stage filtration can have the physical characteristics described above for filtration, i.e., made of synthetic or inorganic material, or material not contaminated with nuclear components, autoclavable, strong, and malleable.

After centrifugation or filtration, the supernatant can be precipitated with, e.g., isopropanol (IPA), and stored at -20°C until chromatography or optionally used immediately for chromatography.

### Anion Exchange Chromatography

Ion exchange chromatography is performed to separate plasmid DNA molecules from contaminating molecules based on molecular ionic charge or isoelectric point (pI) at a given pH. Ion exchange columns can be packed with positively charged beads (for anion exchangers) or negatively charged beads (for cation exchangers) that make up the ion exchange material. The charge density and pI of the molecules will determine the ionic capacity of the support matrix that is suitable for separating the molecules.

The present invention makes use of anion exchange chromatography (AEX). In one embodiment, the AEX chromatography material contains a ceramic matrix. By a ceramic matrix for the AEX is intended to convey use of a chromatography support material using sorbents made of rigid macro-porous ceramic beads ("ceramic matrix"). When such AEX chromatography material contains a ceramic matrix, the ceramic matrix is coated and permeated with functionalized (anionic) hydrogel or resin. Such AEX chromatography material includes, but is not limited to, BioSepra® Ceramic HyperD ion exchanger (Ciphergen Biosystems, Inc., Fremont, CA), e.g., BioSepra® Q Ceramic HyperD. BioSepra® Ceramic HyperD is a high capacity sorbent for efficient and scaleable purification of biomolecules. BioSepra® Ceramic HyperD ion exchangers employ a high-capacity hydrogel polymerized within the pores of a rigid ceramic bead. This design combines the desirable characteristics of a soft, high-capacity hydrogel with the dimensional stability of a rigid ceramic bead. The many ion exchange sites in the hydrogel are highly accessible. Such ceramic matrix-supported AEX gives rise to high dynamic binding capacity is obtained at high linear velocity. Dynamic capacity is the amount of sample that will bind to a gel packed in a column, and run under defined conditions. It depends on pH, ionic strength, counterion, sample, and flow rate. In general, the lower the flow rates, the higher the dynamic capacity. The rigid ceramic matrix of such AEX chromatography material permits higher linear velocity without compression or shrinkage. The beads do not deform under fluid flow. Such AEX chromatography material can be used to increase flow velocity for higher productivity and increase efficiency, and for high purity.

Ceramic matrix also permits increased capacity compared to conventional resins (3 to 4 fold). For example, a single 18cm diameter column has more DNA capacity (5.7g) than a 30cm diameter Q-Sepharose column (5.3g). Large columns are easy to pack because of the nature of the resin (rigid ceramic bead) and packing large columns is routine and not time consuming. The use of such AEX chromatography material affords: (1) reduced processing time; (2) higher flow rate; (3) higher binding capacity; (4) reduced resin and buffer requirements; (5) smaller process equipment requirements; and (6) reduced column costs.

In some aspects of the invention, the average particle diameter of the ceramic matrix for the AEX support (without the hydrogel or resin) can be about 10 µm to about 200 µm. The average pore size of the ceramic matrix for the AEX support (without the hydrogel or resin) can be about 750 Å to about 3000 Å. The average pore size of such AEX chromatography material (the assembled ceramic matrix with the coated hydrogel or resin) can be about 10 Å to about 100 Å.

The sample is loaded onto the AEX column. Alternatively, ion exchange operations can be run using two different mobile phases or buffers (i.e., under gradient conditions). The starting buffer can be a low salt or ionic concentration buffer. The eluting buffer can have a significantly higher ionic concentration than the starting buffer. The operating pH can be determined by sample solubility and support matrix stability. For example, an ion exchanger can be run at a pH of about 6 to about 11 and a linear or step gradient developed between about 0.3 M and about 1.0 M NaCl.

Elution curves can be recorded continuously or determined by investigating individual fractions, by, for example, UV absorbance, agarose gel electrophoresis, and other analytical methods. Fractions containing the DNA product can be pooled.

### Hydrophobic Interaction Chromatography

The ceramic AEX elution peak (pooled or unpooled) is adjusted to a concentration of about 1 M to about 2 M ammonium sulfate, optionally filtered, and then loaded onto a hydrophobicity interaction chromatography (HIC) column such as, e.g., Octyl FF HIC (Amersham Bioscience, Piscataway, NJ), Phenyl FF HIC (Amersham Bioscience, Piscataway, NJ), Butyl FF HIC (Amersham Bioscience, Piscataway, NJ), and Hexyl HIC (Tosoh-Haas, Montgomeryville, PA), for removal or reduction of residual host cell impurities such as endotoxin.

HIC is conducted to resolve molecules based on differences in their surface hydrophobicity. Interactions between hydrophobic groups with hydrophobic ligands attached to a chromatographic matrix mediate this chemistry. The type of matrix, the nature of the hydrophobic groups, and the conditions of absorption and elution can be tailored to suit the unique properties of the molecules involved. A matrix for HIC chromatography material to be used in the invention is, but not limited to, cross-linked agarose resin.

HIC is generally practiced by binding compounds of interest in an aqueous solution containing appropriate concentrations of salt (e.g. ammonium sulfate). Elution of the desired materials is accomplished by lowering the salt concentration. Volatile or flammable organic solvents need not necessarily be used, and no organic modifiers are generally required.

There are a variety of HIC resins which are commercially available, differing in both backbone and functional chemistries. In general, they can be made to work for plasmid DNA purification, such as, but not limited to, Octyl FF HIC (Amersham Bioscience, Piscataway, NJ), Phenyl FF HIC (Amersham Bioscience, Piscataway, NJ), Butyl FF HIC (Amersham Bioscience, Piscataway, NJ), and Hexyl HIC (Tosoh-Haas, Montgomeryville, PA). The HIC step can be a flow through step, i.e., the supercoiled and open circular plasmid flow through the column while RNA, chromosomal DNA, denatured plasmid DNA and endotoxins are retained on the column.

The HIC resins are initially equilibrated in high ionic strength multivalent salts such as ammonium sulfate. These conditions enhance hydrophobic interaction and thus retention of various biomolecules. By reducing the salt concentration the hydrophobic interaction is lessened and the biomolecules begin to elute from the resin depending on their hydrophobicity. Thus, the supercoiled plasmid can be purified from undesired plasmid forms (open circular, denatured) and from contaminants using HIC.

The interaction of biomolecules with the HIC resins can also be modified as a function of the multivalent salt selected. It has been observed that in general sodium citrate and potassium phosphate salts can increase hydrophobic interaction of plasmid DNA greater than ammonium sulfate. Thus, by optimizing the selection of chromatography material and salt conditions, one can affect the quality of purification for a given plasmid construct. Undertaking such optimization is understood and well within the skill in the art.

Elution curves can be recorded continuously or determined by investigating individual fractions, by, for example, UV absorbance, agarose gel electrophoresis, and other analytical methods. Fractions containing the DNA product can be pooled.

The Octyl HIC polishing step binds trace contaminants (not product), therefore a substantially smaller column is required compared to size exclusion chromatography (SEC). Because the HyperD elution peak is immediately loaded onto the HIC column for polishing, the entire chromatography process train is completed in a single eight-hour shift. The overnight alcohol precipitation step to concentrate the DNA post Q-Sepharose pre SEC is eliminated (necessary in the current process).

The use of HIC as required in the process of the invention provides significant increase in throughput capacity, reduction in column size required to process equivalent amount of product, significant reduction in process time (e.g., higher flow rate than SEC), scaleability, reduced consumption of support materials (e.g., reduced resin and buffer requirements), and reduced production costs.

### Further Processing

The DNA product obtained by the process described above can be further concentrated by ultrafiltration. The DNA product can be further diafiltered to remove ammonium sulfate. The DNA product can be further precipitated with ethanol.

The process of the invention can be performed in the absence of any added enzymes, organic extracts, and mutagenic reagents.

### DNA Product

In some aspects, the invention is directed to a DNA product obtained by the purification process of the invention. The DNA product obtained by the process of the invention can be used directly or further purified or processed. The DNA product of the invention can contain about 95% or greater by weight of circular plasmid DNA. The DNA product can contain less than about 5% by weight of RNA, such as, e.g., about 5% by weight of RNA to about 0.00001%, or less than 0.0001%. The DNA product can contain less than about 0.002 µg of host DNA/µg of DNA product, such as, e.g., about 0.002 µg of host DNA/µg of DNA product to about 0.00002 µg of host DNA/µg of DNA product, or less than 0.00004 µg of host DNA/µg of plasmid DNA. The DNA product can contain less than about 0.001 µg protein/µg of DNA product, such as about 0.001 µg protein/µg of DNA product to about 0.00000001 µg protein/µg of DNA product. The DNA product can contain less than about 0.01 EU/µg of DNA product, such as, e.g., about 0.0001 EU/µg to about 0.0002 EU/µg (EU: endotoxin units; measurement of endotoxin was accomplished according to USP File<85>). The DNA product obtained by the process of the invention can comprise about 95% or greater by weight of circular plasmid DNA, wherein said DNA product contains less than about 5% by weight of RNA, less than about 0.002 µg of host DNA/µg of DNA product, less than about 0.001 µg of protein/µg of DNA product, and less than about 0.01 EU/µg of DNA product.

In some aspects, the invention is directed to a DNA product comprising about 95% or greater by weight of circular plasmid DNA, wherein said DNA product contains less than about 5% by weight of RNA, less than about 0.002 µg of host DNA/µg of DNA product, less than about 0.001 µg of protein/µg of DNA product, and less than about 0.01 EU/µg of DNA product. The DNA product contains less than about 5% by weight of RNA, such as, e.g., about 5% by weight of RNA to about 0.00001%, or less than 0.0001%. The DNA product can contain less than about 0.002 µg of host DNA/µg of DNA product, such as, e.g., about 0.002 µg of host DNA/µg of DNA product to about 0.00002 µg of host DNA/µg of DNA product, or less than 0.00004 µg of host DNA/µg of plasmid DNA. The DNA product can contain less than about 0.001 µg protein/µg of DNA product, such as about 0.001 µg protein/µg of DNA product to about 0.00000001 µg protein/µg of DNA product. The DNA product can contain less than about 0.01 EU/µg of DNA product, such as, e.g., about 0.0001 EU/µg to about 0.0002 EU/µg (EU: endotoxin units; measurement of endotoxin was accomplished according to USP File<85>).

Methods for measuring levels of DNA, RNA, protein or endotoxins are known in the art. For example, endotoxin levels can be measured by LAL assay. Host cell chromosomal DNA levels can be measured by Southern blot assay or chromatographically, such as by HPLC. Protein can be measured by colorimetric analysis such as BCA and amino acid analysis such as used in protein sequencing. RNA can be measured by Northern blot assay, ethidium bromide agarose analysis, or chromatographically, such as by HPLC.

### Pharmaceutical Grade Plasmid DNA

Upon obtaining the DNA product of the process of the invention, the DNA product can be used as, but not limited to, a medicament as the process of the invention can provide a pharmaceutical grade DNA product. The DNA product can be diluted in formulation buffer, such lactated Ringer's, 0.9% saline or PBS injection vehicle or other innocuous buffered delivery vehicle, or it can be precipitated or concentrated and then brought up in formulation buffer using known techniques as will be recognized by those with skill in the art.

At this stage, a solution containing pharmaceutical grade plasmid DNA can be sterilized. Any of several sterilization techniques can be adapted. For example, sterilization can be achieved by filtration.

A filter material can be implemented that is characterized by pores having a diameter large enough to allow the filtrate to pass through efficiently but small enough so that the filter material retains microorganisms and the like. Accordingly pore sizes can be from about 0.01 µm to about 10 µm, such as, e.g., from about 0.1 µm to about 1 µm. The material from which the filter is manufactured can be a synthetic material or an organic or inorganic natural material; however, it should tend not to bind plasmid DNA. Nor should it be inclined to restrict substances the are meant to flow through the filter along with the plasmid DNA, e.g., ingredients composing formulation buffers and such. Additionally, it should be non-pyrogenic. In some embodiments, the filter material has an average pore size of about 0.2 µm. The filter can also be sterile.

Sterilization is advantageously conducted under aseptic conditions in a class 100 hood or equivalent. It is contemplated that plasmid DNA can be precipitated or concentrated either before or after sterile filtration and resuspended in a formulation buffer. Sterilization can be conducted before or after the DNA product is formulated.

Formulating agents for the medicament include, but are not limited to, Ca phosphate, viral protein, lipophilic agents, any material that aids the DNA delivery, or any material that enhances the biological activity of the medicament.

### Transfection Facilitating Materials

Formulations of the DNA product of the present invention can also include one or more transfection facilitating materials that facilitate delivery of polynucleotides to the interior of a cell, and/or to a desired location within a cell. Examples of the transfection facilitating materials include, but are not limited to lipids, such as cationic lipids; inorganic materials such as calcium phosphate, and metal (*e.g*., gold or tungsten) particles (*e.g*., "powder" type delivery solutions); peptides, including cationic peptides, targeting peptides for selective delivery to certain cells or intracellular organelles such as the nucleus or nucleolus, and amphipathic peptides, *i.e*. helix forming or pore forming peptides; basic proteins, such as histones; asialoproteins; viral proteins (*e.g*., Sendai virus coat protein); pore-forming proteins; and polymers, including dendrimers, star-polymers, "homogenous" poly-amino acids (*e.g*., poly-lysine, poly-arginine), "heterogenous" poly-amino acids (*e.g*., mixtures of lysine & glycine), co-polymers (*e.g*., block co-polymers and poloxamers), polyvinylpyrrolidinone (PVP), and polyethylene glycol (PEG).

A transfection facilitating material can be used alone or in combination with one or more other transfection facilitating materials. Two or more transfection facilitating materials can be combined by chemical bonding (*e.g*., covalent and ionic such as in lipidated polylysine, PEGylated polylysine) (Toncheva, V., et al., Biochim. Biophys. Acta 1380(3):354-368 (1998)), mechanical mixing (*e.g*., free moving materials in liquid or solid phase such as "polylysine + cationic lipids") (Gao, X., and Huang, L., Biochemistry 35:1027-1036 (1996); Trubetskoy, V.S., et al., Biochem. Biophys. Acta 1131:311-313 (1992)), and aggregation (*e.g*., co-precipitation, gel forming such as in cationic lipids + poly-lactide co-galactide, and polylysine + gelatin).

The invention includes a composition or medicament comprising the DNA product of the invention and an auxiliary agent, which is administered before, after, or concurrently with the DNA product. As used herein, an "auxiliary agent" is a substance included in a composition for its ability to enhance, relative to a composition which is identical *except* for the inclusion of the auxiliary agent, the entry of plasmid into mammalian cells *in vivo*, and/or the *in vivo* expression of polypeptides encoded by such polynucleotides. Certain auxiliary agents may, in addition to enhancing entry of polynucleotides into cells, enhance an immune response to an immunogen encoded by the polynucleotide. Auxiliary agents of the present invention include nonionic, anionic, cationic, or zwitterionic surfactants or detergents, such as nonionic surfactants or detergents, chelators, DNase inhibitors, poloxamers, agents that aggregate or condense nucleic acids, emulsifying or solubilizing agents, wetting agents, gel-forming agents, and buffers.

In certain embodiments of the invention, the composition or medicament comprising the DNA product of the invention includes an adjuvant which is administered before, after, or concurrently with the plasmid. A great variety of materials have been shown to have adjuvant activity through a variety of mechanisms. Any compound which can increase the expression, antigenicity or immunogenicity of a polypeptide encoded by the plasmid is a potential adjuvant. Examples of various adjuvants include, but are not limited to: inert carriers, such as alum, bentonite, latex, and acrylic particles; pluronic block polymers, such as TiterMax^{™}; depot formers, such as Freunds adjuvant, surface active materials, such as saponin, lysolecithin, retinal, Quil A, liposomes, and pluronic polymer formulations; macrophage stimulators, such as bacterial lipopolysaccharide; alternate pathway complement activators, such as insulin, zymosan, endotoxin, and levamisole; and non-ionic surfactants, such as poloxamers, poly(oxyethylene)-poly(oxypropylene) triblock copolymers; and cytokines, such as GM-CSF and IL-12.

### Pharmaceutical Kits

It is additionally contemplated that, in conclusion, the final formulated DNA product can be filled into containers such as, e.g., glass container, plastic container, vial, test tube, flask, bottle, syringe, or other container means, or strips of plastic or paper, into which the DNA product and any other desired agent can be placed, and can be suitably aliquoted. Where additional components are included, it will also generally contain a second vial or other container into which these are placed, enabling the administration of separated designed doses. Such kits can also comprise a second/third container means for containing a sterile, pharmaceutically acceptable buffer or other diluent. The kit can also contain a means by which to administer a medicament comprising the DNA product of the invention to an animal or patient, e.g., one or more needles or syringes, or even an eye dropper, pipette, or other such like apparatus, from which the formulation can be injected into the animal or applied to a diseased area of the body. The kits of the present invention will also typically include a means for containing the containers such as vials, or such like, and other components, in close confinement for commercial sale, such as, e.g., injection or blow-molded plastic containers into which the desired containers and other apparatus are placed and retained. In some embodiments, the kits of the invention can include in close confinement a means for containing the formulated DNA material in one vial and a means for another vial with another formulating agent (e.g., carrier, diluent, excipient, transfection facilitating material) or another pharmaceutically active agent, which can be combined before use.

Each of the pharmaceutical kits can further comprise an administration means. Means for administration include, but are not limited to syringes and needles, catheters, biolistic injectors, particle accelerators, i.e., "gene guns," pneumatic "needleless" injectors, gelfoam sponge depots, other commercially available depot materials, e.g., hydrogels, osmotic pumps, and decanting, polynucleotide coated sutures, skin patches, or topical applications during surgery.

Each of the pharmaceutical kits can further comprise an instruction sheet for administration of the composition to a mammal. The plasmid components of the composition can be provided as a liquid solution or they can be provided in lyophilized form as a dried powder or a cake. If the plasmid is provided in lyophilized form, the dried powder or cake can also include any salts, auxiliary agents, transfection facilitating agents, and additives of the composition in dried form. Such a kit can further comprise a container with an exact amount of sterile pyrogen-free water, for precise reconstitution of the lyophilized components of the kit.

The container in which the plasmid or other kit components is packaged prior to use can comprise a hermetically sealed container enclosing an amount of the lyophilized formulation or a solution containing the formulation suitable for a pharmaceutically effective dose thereof, or multiples of an effective dose. The kit components typically are packaged in a sterile container, and the hermetically sealed container is designed to preserve sterility of the pharmaceutical formulation until use. Optionally, the container can be associated with administration means and/or instruction for use.

All of the various aspects, embodiments and options described herein can be combined in any and all variations.

Particular aspects of the invention can be more readily understood by reference to the following examples, which are intended to exemplify the invention, without limiting its scope to the particular exemplified embodiments.

### EXAMPLES

### EXAMPLE 1

### Bacterial cell lysis and plasmid recovery

One embodiment of the process is outlined in Figure 1.

Step (a) alkaline lysis. Approximately 800 grams of E. *coli* cells containing a 6800 bp plasmid DNA derived from pBR322 were lysed using a modification of the alkaline lysis method. Cell resuspension, lysis, and neutralization were accomplished in a 30L ASME 316L stainless steel pressure vessel using a 1" ID 12 element static mixer and peristaltic pump in a continuous re-circulation mode. Cell resuspension, lysis and neutralization were accomplished at room temperature in approximately 45 min.

Lysate clarification (step b) was accomplished by adding approximately 720 grams of Celite SuperCel HyFlo (acid washed) to the lysate, mixing, and then pressure feeding the mixture through a 12-inch, 9-cell Cuno SP60 depth filter (Cuno Inc., Meriden, CT). Post clarification, the depth filter was flushed with 4L of TE (10 mM TRIS, 1 mM EDTA, pH 8.0) to maximize recovery.

The clarified lysate was then ultrafiltered and diafiltered (step c) using a 300kD NMWC hollow fiber system to reduce the volume and exchange the lysis solution to a buffered solution by TFF (300 kD hollow fiber membrane; A/G Technology Corp., Needham, MA). Volume was reduced 10-15 fold followed by a 4 volume buffer exchange.

In step (d), the concentrated lysate was selectively precipitated using polyethylene glycol (PEG-8000) at a final concentration of 10% in 0.53M NaCl for further DNA enrichment. The DNA precipitant was recovered by centrifugation (step e) then dissolved in TE buffer (step f). When fully dissolved, ammonium acetate (step g) was added to the solution to a final concentration of 2.5M to selectively precipitate contaminants.

The contaminants were removed by centrifugation; the DNA containing supernatant was precipitated with cold isopropanol for storage until chromatography.

### Chromatography

Approximately 60mg of the IPA precipitated DNA was removed and recovered by centrifugation. The DNA pellet was dissolved in 50mM Tris pH 8, 1mM EDTA, 0.65M NaCl, pH 8.0.

A 1.6 x 12cm column was packed with Q Ceramic HyperD, a strong anion exchange resin. The column was equilibrated with Buffer A (50mM Tris pH 8, 1mM EDTA, 0.65M NaCl, pH 8.0) (linear flow rate = 150cm/hr). After loading the sample (step h), the column was washed with 5 CV (column volumes) of 0% buffer B (50mM Tris pH 8, 1mM EDTA, 1M NaCl, pH 8.0), then stepped to 6% buffer B for an additional 5 CV, then stepped to 100% buffer B to elute the purified DNA.

The final polishing chromatography step (i) was accomplished using Octyl Sepharose Fast Flow resin. This step can reduce residual levels of endotoxin and host cell chromosomal DNA by binding the contaminants while the plasmid passes through the column.

The HyperD elution peak was precipitated and the DNA pellet dissolved in 2M ammonium sulfate, 50mM Tris, 1mM EDTA, pH 8.0, then loaded onto a 2.6 x 2.5cm column equilibrated in the same buffer (linear flow rate = 75cm/hr). The flow-through peak containing the final purified plasmid DNA was collected and dialyzed to remove salts.

Quality analysis of the plasmid DNA showed endotoxin levels of 0.0002EU/µg DNA product by LAL Assay and host cell chromosomal DNA levels of 0.0004µg/µg DNA product by Southern Blot Assay. Percent supercoiled was 95% by HPLC analysis.

### EXAMPLE 2

Description, construction and culturing of VCL-1005 are described in U.S. Patent No. 5,561,064. Frozen *E. coli* cells containing VCL-1005 plasmid DNA (4900bp) were resuspended in 25mM Tris-HCl, 10mM EDTA, and 55mM glucose, pH 8.0. Lysis occurred after the addition of 0.2M NaOH, 1% SDS and incubation for 8 minutes. Precipitation of cellular debris occurred by adding 3M potassium acetate, pH 5.5, and 8 additional minutes of incubation. Removal of cellular debris and volume reduction were accomplished by diatomite aided depth filtration followed by ultrafiltration and diafiltration.

The plasmid DNA was PEG precipitated followed by ammonium acetate precipitation to further reduce contaminants. The final plasmid containing supernatant was IPA precipitated for storage until anion exchange chromatography.

IPA precipitated plasmid DNA purified by Q-Sepharose anion exchange chromatography was collected by centrifugation. The DNA pellet was dissolved in 2M ammonium sulfate buffer then loaded onto a 2.6 x 2.5cm Octyl HIC column equilibrated in the same buffer. The flow through fraction was collected and dialyzed against TE buffer to remove the salts. After completion of dialysis, host cell DNA levels were 0.00025µg host cell DNA/µg DNA product and endotoxin levels were 0.0002EU/µg DNA product.

### EXAMPLE 3

Frozen *E. coli* cells containing a 6800 bp plasmid DNA containing portions of pBR322 were resuspended in 25mM Tris-HCl, 10mM EDTA, and 55mM glucose, pH 8.0. Lysis occurred after the addition of 0.2M NaOH, 1% SDS and incubation for 8 minutes. Precipitation of cellular debris occurred by adding 3M potassium acetate, pH 5.5, and 8 additional minutes of incubation. Removal of cellular debris and volume reduction was accomplished by diatomite aided depth filtration followed by ultrafiltration and diafiltration.

The plasmid DNA was PEG precipitated followed by ammonium acetate precipitation to further reduce contaminants. The final plasmid containing supernatant was IPA precipitated for storage until anion exchange chromatography.

IPA precipitated DNA purified by Q Ceramic HyperD anion exchange chromatography was collected by centrifugation. The DNA pellet was dissolved in 2M ammonium sulfate buffer then loaded onto a 2.6 x 2.5cm Octyl HIC column equilibrated in the same buffer. The flow through fraction was collected and dialyzed against TE buffer to remove the salts. After completion of dialysis, host cell DNA levels were 0.00029µg/µg DNA product, and endotoxin levels were 0.00001EU/µg DNA product.

### EXAMPLE 4

Frozen *E. coli* cells containing VCL-1005 plasmid DNA (4900bp) were resuspended in 25mM Tris-HCl, 10mM EDTA, and 55mM glucose, pH 8.0. Lysis occurred after the addition of 0.2M NaOH, 1% SDS and incubation for 8 minutes. Precipitation of cellular debris occurred by adding 3M potassium acetate pH 5.5 and 8 additional minutes of incubation. Removal of cellular debris and volume reduction were accomplished by diatomite aided depth filtration followed by ultrafiltration and diafiltration.

The plasmid DNA was PEG precipitated followed by ammonium acetate precipitation to further reduce contaminants. The final plasmid containing supernatant was IPA precipitated for storage until anion exchange chromatography.

DNA purified by Q Ceramic HyperD anion exchange chromatography was diluted 1:1 with 4M ammonium sulfate buffer then loaded onto a 2.6 x 2.5cm Octyl HIC column equilibrated in 2M ammonium sulfate buffer. The flow through fraction was collected and dialyzed against TE buffer to remove the salts. After completion of dialysis, host cell DNA levels were 0.001µg host cell DNA/µg DNA product, and endotoxin levels were 0.0006EU/µg DNA product.

### EXAMPLES

Frozen *E. coli* cells containing a 6694 bp plasmid DNA were resuspended in 25mM Tris-HCl, 10mM EDTA, and 55mM glucose, pH 8.0. Lysis occurred after the addition of 0.2M NaOH, 1% SDS and incubation for 8minutes. Precipitation of cellular debris occurred by adding 3M potassium acetate, pH 5.5, and 8 additional minutes of incubation. Removal of cellular debris and volume reduction was accomplished by diatomite aided depth filtration followed by ultrafiltration and diafiltration.

The plasmid DNA was PEG precipitated followed by ammonium acetate precipitation to further reduce contaminants. The final plasmid containing supernatant was IPA precipitated for storage until anion exchange chromatography.

IPA precipitated DNA purified by Q-Sepharose anion exchange chromatography was collected by centrifugation. The DNA pellet was dissolved in 2M ammonium sulfate buffer then loaded onto a 2.6 x 2.5cm Octyl HIC column equilibrated in the same buffer. The flow through fraction was collected and dialyzed against TE buffer to remove the salts. After completion of dialysis, host cell DNA levels were 0.0002µg host cell DNA/µg DNA product, and endotoxin levels were 0.01EU/µg DNA product.

### EXAMPLE 6

### Large-scale bacterial cell lysates and plasmid recovery

720 grams of frozen *E. Coli* cells containing VR-4700 plasmid DNA (6400bp) (Sankar, V. *et al., Oral Diseases 8*:275-281 (2002)) was resuspended in 4,320mL of 25mM Tris-HCl, 10mM EDTA, and 55mM glucose in a 30L ASME 316L pressure vessel using a 1" ID 12 element static mixer and peristaltic pump in a continuous re-circulation mode. Lysis occurred after the addition of 8,640mL of 0.2M NaOH, 1% SDS which re-circulated for 8 minutes. Neutralization and precipitation of cellular debris occurred by adding 6,480mL of 3M potassium acetate pH 5.5, which re-circulated for an additional 8 minutes.

Lysate clarification was accomplished by adding 720 grams of Celite SuperCel HyFlo (acid washed) to the said lysate, mixing, then pressure feeding (10psi) the mixture through a 12-inch, 9-cell Cuno SP60 depth filter (Cuno Inc., Meriden, CT). Post clarification, the depth filter was flushed with 4L of 10mM Tris pH 8.0, 1mM EDTA to maximize recovery.

The clarified lysate was ultrafiltered using a 300kD NMWC hollow fiber system to reduce the volume 10 fold followed by a 4-volume diafiltration with 10mM Tris pH 8.0, 1mM EDTA to exchange the lysis solution to a buffered solution. After the final diafiltration, the retentate was collected in a depyrogenated precipitation vessel. The membrane was then rinsed with a minimal volume of diafiltration buffer to maximize recovery; the rinse was added to the retentate.

The concentrated lysate was selectively precipitating using polyethylene glycol (PEG-8000) at a final concentration of 10% in 0.53M NaCl for further DNA enrichment. The DNA precipitant was recovered by centrifugation (6500 x g for 30min.) then dissolved in TE buffer using 1mL/gram of starting cell paste. When fully dissolved, ammonium acetate was added to the solution to a final concentration of 2.5M to selectively precipitate contaminants. The contaminants were recovered by centrifugation (6500 x g for 30min.). The plasmid containing supernatant was precipitated with an equal volume of IPA and stored at -20°C until anion exchange chromatography.

### Chromatography

1000mg of the IPA precipitated DNA was collected by centrifugation. The pellet was dissolved in 50mM Tris pH 8, 1mM EDTA, 0.65M NaCl to a final concentration of 2mg/mL (± 0.2). The dissolved DNA pellet was then passed through a 0.45µ filter.

A 7 x 18cm Millipore QuikScale column was packed with Q Ceramic HyperD, a strong anion exchange resin. The column was equilibrated with 50mM Tris, pH 8, 1mM EDTA, 0.65M NaCl at a linear flow rate of 150cm/hr. After loading the sample, the column was washed with 5 CV of 0% buffer B (50mM Tris pH 8, 1mM EDTA, 1M NaCl), then stepped to 6% buffer B for an additional 5 CV, the flow rate was reduced to 60cm/hr before stepping to 100% buffer B to elute the purified pDNA. The collected HyperD elution peak was then diluted 1:1 with 3M ammonium sulfate.

The final polishing chromatography step was accomplished using Octyl Sepharose Fast Flow, a hydrophobic interaction resin. A 5 x 10cm Amersham Bioscience column was packed with Octyl Sepharose Fast Flow resin. The column was equilibrated with 5 CV of 1.5M ammonium sulfate, 50mM Tris pH 8, 1mM EDTA at a linear flow rate of 75cm/hr. Following equilibration, the diluted HyperD elution peak was loaded onto the column at a linear flow rate of 75cm/hr. The flow-through peak containing final purified plasmid DNA was collected then concentrated 10-fold using a 300K NMWC hollow fiber ultrafiltration system. After concentrating, diafiltration was performed using 10mM Tris pH 8.0, 1mM EDTA, and 150mM NaCl to remove the ammonium sulfate. UF Permeate conductivity was checked to confirm ammonium sulfate removal. After the final diafiltration, the retentate was collected in a sterile vessel. The membrane was then rinsed with a minimal volume of diafiltration buffer; the rinse was added to the retentate to maximize recovery.

Quality analyses of plasmid DNA showed endotoxin levels of 0.00019EU/µg of pDNA by LAL analysis. Host cell DNA levels were 0.0002µg host cell DNA/µg pDNA by southern analysis. Supercoiled DNA content was 92% by HPLC analysis.

Four additional large-scale processes were completed using VCL-1005 plasmid DNA (4900bp). Quality analysis from these plasmid DNA processes resulted in a mean residual endotoxin level of 0.0001EU/µg (std. dev. = 0.0001). Host cell DNA levels averaged 0.0005µg/µg (std. dev. = 0.0003). Supercoiled DNA content averaged about 90%.

One skilled in the art would readily appreciate that the present invention is well adapted to carry out the objects and obtain the ends and advantages mentioned, as well as those inherent therein. The methods, procedures, treatments, molecules, specific compounds described herein are presently representative embodiments and are not intended as limitations on the scope of the invention. Changes therein and other uses will occur to those skilled in the art.

## Claims

1. A process for purifying plasmid DNA from host cell impurities to obtain a DNA product, said process comprising:
(a) lysing host cells containing the plasmid DNA to obtain a lysate;
(b) clarifying said lysate by depth filtration to obtain a clarified lysate;
(c) ultrafiltering said clarified lysate to obtain an ultrafiltered clarified lysate;
(d) adding a first precipitating agent in sufficient quantity to said ultrafiltered clarified lysate to obtain a precipitate of the plasmid DNA;
(e) dissolving said precipitate to obtain a first solution;
(f) adding a second precipitation agent in sufficient quantity to said solution to precipitate the host cell impurities and to obtain a solute containing the plasmid DNA;
(g) transferring said solute into another buffer to obtain a second solution;
(h) applying said second solution to an anion exchange chromatography (AEX) material to obtain an eluate containing the plasmid DNA; and
(i) applying said eluate to a hydrophobic interaction chromatography (HIC) material to obtain the DNA product.

2. The process of claim 1, wherein said AEX material comprises a ceramic matrix.

3. The process of claim 2, wherein an average particle diameter of said ceramic matrix is about 10 µm to about 200 µm.

4. The process of claim 2, wherein an average pore size of said ceramic matrix is about 750 Å to about 3000 Å.

5. The process of claim 2, wherein an average pore size of said AEX material is about 10 Å to about 100 Å.

6. The process in claim 1, wherein an average particle diameter of resin for said HIC material is about 50 µm to about 150 µm.

7. The process in claim 1, wherein an average pore size of resin for said HIC material is about 25 nm to about 100 nm.

8. The process claim of 1, wherein said lysing in (a) is by alkaline lysis.

9. The process of claim 1, wherein said clarifying in (b) is by diatomite aided depth filtration.

10. The process of claim 1, wherein said ultrafiltering in (c) is by hollow fiber ultrafiltration.

11. The process of claim 1, wherein said first precipitating agent in (d) is polyethylene glycol (PEG).

12. The process of claim 1, wherein said second precipitating agent in (f) is ammonium acetate.

13. The process of claim 1, wherein said eluate in (h) is adjusted to a concentration of about 1M to about 2M ammonium sulfate.

14. The process of claim 1, wherein said HIC material in (i) contains cross-linked agarose resin.

15. The process of claim 1, further comprising concentrating said DNA product in (i) by ultrafiltration.

16. The process of claim 1, further comprising diafiltering said DNA product in (i) to remove ammonium sulfate.

17. The process of claim 1, wherein said DNA product is precipitated with ethanol.

18. The process of claim 1, which is conducted in the absence of any added enzymes, organic extractants, or mutagenic reagents.

19. The process of claim 1, further comprising sterilizing, formulating, and filling in a sterile container said DNA product.

20. The process of claim 1, wherein said host cells are bacteria.

## Patentansprüche

1. Verfahren zur Reinigung von Plasmid-DNA von Wirtszellen-Verunreinigungen, um ein DNA-Produkt zu erhalten, wobei das Verfahren Folgendes umfasst:
(a) das Lysieren von Wirtszellen, welche die Plasmid-DNA enthalten, um ein Lysat zu erhalten;
(b) das Klären des Lysats durch Tiefenfiltration, um ein geklärtes Lysat zu erhalten;
(c) das Ultrafiltrieren des geklärten Lysats, um ein ultrafiltriertes geklärtes Lysat zu erhalten;
(d) das Hinzufügen eines ersten Fällungsmittels in ausreichender Menge zu dem ultrafiltrierten geklärten Lysat, um ein Präzipitat der Plasmid-DNA zu erhalten;
(e) das Auflösen des Präzipitats, um eine erste Lösung zu erhalten;
(f) das Hinzufügen eines zweiten Fällungsmittels in ausreichender Menge zu der Lösung, um die Wirtszellen-Verunreinigungen zu fällen und um einen gelösten Stoff zu erhalten, der die Plasmid-DNA erhält;
(g) das Transferieren des gelösten Stoffs in einen anderen Puffer, um eine zweite Lösung zu erhalten;
(h) das Aufbringen der zweiten Lösung auf ein Anionenaustausch-Chromatographie- (AEX-) Material, um ein Eluat zu erhalten, das die Plasmid-DNA enthält; sowie
(i) das Aufbringen des Eluats auf ein Hydrophobchromatographie- (HIC-) Material, um das DNA-Produkt zu erhalten.

2. Verfahren nach Anspruch 1, worin das AEX-Material eine keramische Matrix umfasst.

3. Verfahren nach Anspruch 2, worin ein durchschnittlicher Teilchendurchmesser der keramischen Matrix etwa 10 µm bis etwa 200 µm beträgt.

4. Verfahren nach Anspruch 2, worin eine durchschnittliche Porengröße der keramischen Matrix etwa 750 Å bis etwa 3000 Å beträgt.

5. Verfahren nach Anspruch 2, worin eine durchschnittliche Porengröße des AEX-Materials etwa 10 Å bis etwa 100 Å beträgt.

6. Verfahren nach Anspruch 1, worin ein durchschnittlicher Harz-Teilchendurchmesser für das HIC-Material etwa 50 µm bis etwa 150 µm beträgt.

7. Verfahren nach Anspruch 1, worin eine durchschnittliche Harz-Porengröße für das HIC-Material etwa 25 nm bis etwa 100 nm beträgt.

8. Verfahren nach Anspruch 1, worin das Lysieren in (a) durch alkalische Lyse erfolgt.

9. Verfahren nach Anspruch 1, worin das Klären in (b) durch eine Diatomit-unterstützte Tiefenfiltration erfolgt.

10. Verfahren nach Anspruch 1, worin das Ultrafiltrieren in (c) durch eine Hohlfaser-Ultrafiltration erfolgt.

11. Verfahren nach Anspruch 1, worin das erste Fällungsmittel in (d) Polyethylenglykol (PEG) ist.

12. Verfahren nach Anspruch 1, worin das zweite Fällungsmittel in (f) Ammoniumacetat ist.

13. Verfahren nach Anspruch 1, worin das Eluat in (h) auf eine Konzentration von etwa 1 M bis etwa 2 M Ammoniumsulfat eingestellt ist.

14. Verfahren nach Anspruch 1, worin das HIC-Material in (i) vernetztes Agarose-Harz enthält.

15. Verfahren nach Anspruch 1, weiters umfassend das Einengen des DNA-Produkts in (i) durch Ultrafiltration.

16. Verfahren nach Anspruch 1, weiters umfassend das Diafiltrieren des DNA-Produkts in (i), um Ammoniumsulfat zu entfernen.

17. Verfahren nach Anspruch 1, worin das DNA-Produkt mit Ethanol gefällt wird.

18. Verfahren nach Anspruch 1, das in Abwesenheit etwaiger hinzugefügter Enzyme, organischer Extraktionsmittel oder mutagener Reagenzien durchgeführt wird.

19. Verfahren nach Anspruch 1, weiters umfassend das Sterilisieren und Formulieren des DNA-Produkts sowie das Füllen desselben in einen sterilen Behälter.

20. Verfahren nach Anspruch 1, worin die Wirtszellen Bakterien sind.

## Revendications

1. Procédé de purification d'ADN plasmidique à partir d'impuretés de cellules hôtes pour obtenir un produit d'ADN, ledit procédé comprenant les étapes consistant à:
(a) lyser des cellules hôtes contenant l'ADN plasmidique pour obtenir un lysat;
(b) clarifier ledit lysat par une filtration en profondeur pour obtenir un lysat clarifié;
(c) ultra-filtrer ledit lysat clarifié pour obtenir un lysat clarifié ultra-filtré;
(d) ajouter un premier précipitant en quantité suffisante audit lysat clarifié ultra-filtré pour obtenir un précipité d'ADN plasmidique;
(e) dissoudre ledit précipité pour obtenir une première solution;
(f) ajouter un deuxième précipitant en quantité suffisante à ladite solution pour précipiter les impuretés de cellules hôtes et pour obtenir un soluté contenant l'ADN plasmidique;
(g) transférer ledit soluté dans un hôte tampon pour obtenir une deuxième solution;
(h) appliquer ladite deuxième solution à un matériau de chromatographie d'échange d'anions AEX pour obtenir un éluat contenant l'ADN plasmidique; et
(i) appliquer ledit éluat à un matériau de chromatographie d'interaction hydrophobe (HIC) pour obtenir le produit d'ADN.

2. Procédé selon la revendication 1, où ledit matériau AEX comprend une matrice céramique.

3. Procédé selon la revendication 2, où un diamètre de particules moyen de ladite matrice céramique est d'environ 10 µm à environ 200 µm.

4. Procédé selon la revendication 2, où une taille de pore moyenne de ladite matrice céramique est d'environ 750 Å à environ 3000 Å.

5. Procédé selon la revendication 2, où une taille de pore moyenne dudit matériau AEX est d'environ 10 Å à environ 100 Å.

6. Procédé selon la revendication 1, où un diamètre de particules moyen d'une résine pour ledit matériau HIC est d'environ 50 µm à environ 150 µm.

7. Procédé selon la revendication 1, où une taille de pore moyenne de la résine pour ledit matériau HIC est d'environ 25 nm à environ 100 nm.

8. Procédé selon la revendication 1, où ledit lysage dans (a) se fait par lyse alcaline.

9. Procédé selon la revendication 1, où ladite clarification dans (b) se fait par filtration en profondeur à l'aide de diatomite.

10. Procédé selon la revendication 1, où ladite ultra-filtration dans (c) se fait par ultra-filtration à fibres creuses.

11. Procédé selon la revendication 1, où ledit premier précipitant dans (d) est du polyéthylène glycol (PEG).

12. Procédé selon la revendication 1, où ledit deuxième précipitant dans (f) est de l'acétate d'ammonium.

13. Procédé selon la revendication 1, où ledit éluat dans (h) est réglé à une concentration d'environ 1M à environ 2M de sulfate d'ammonium.

14. Procédé selon la revendication 1, où ledit matériau HIC dans (i) contient de la résine d'agarose réticulée.

15. Procédé selon la revendication 1, comprenant en outre la concentration dudit produit d'ADN dans (i) par ultra-filtration.

16. Procédé selon la revendication 1, comprenant en outre la diafiltration dudit produit d'ADN dans (i) pour retirer le sulfate d'ammonium.

17. Procédé selon la revendication 1, où ledit produit d'ADN est précipité avec de l'éthanol.

18. Procédé selon la revendication 1, qui est effectué en l'absence de toute enzyme ajoutée, agents d'extraction organiques ou réactifs mutagéniques.

19. Procédé selon la revendication 1, comprenant en outre la stérilisation, la formulation et l'introduction dans un contenant stérile dudit produit d'ADN.

20. Procédé selon la revendication 1, où lesdites cellules hôtes sont des bactéries.
